# EUROPÄISCHE PATENTSCHRIFT

(11) **EP 3 462 459 B1**
(45) Veröffentlichungstag und Bekanntmachung des Hinweises auf die Patenterteilung: **13.11.2024**
(21) Anmeldenummer: 17194117.2
(22) Anmeldetag: 29.09.2017
(51) Int. Cl.: G16H 30/40, G16H 30/20

(54) **CINEMATIC RENDERING KURZFILM ZUR VERANSCHAULICHUNG EINES KLINISCHEN BEFUNDES**
CINEMATIC RENDERING OF SHORT FILM FOR VISUALIZING A CLINICAL DECISION
FILM COURT CINÉMATIQUE DE RENDU PERMETTANT D'ILLUSTRER UN RÉSULTAT CLINIQUE

(43) Veröffentlichungstag der Anmeldung: 03.04.2019
(73) Patentinhaber: Siemens Healthineers AG, 91301 Forchheim (DE)
(72) Erfinder: Nolte, Björn, 90765 Fürth (DE)
(74) Vertreter: Siemens Healthineers Patent Attorneys

(56) Entgegenhaltungen:
- WO-A1-03/071779
- DAPPA EVELYN ET AL: "Cinematic rendering - an alternative to volume rendering for 3D computed tomography imaging", INSIGHTS INTO IMAGING, vol. 7, no. 6, 15 September 2016 (2016-09-15), pages 849 - 856, XP055786984, Retrieved from the Internet <URL:http://link.springer.com/article/10.1007/s13244-016-0518-1/fulltext.html> DOI: 10.1007/s13244-016-0518-1
- MOSER SUSANNE ELISABETH: "Körperkino für das Tumorboard", DEUTSCHES ÄRTZEBLATT, 4 September 2017 (2017-09-04), pages 1594 - 1596, XP055787042, Retrieved from the Internet <URL:https://cdn.aerzteblatt.de/pdf/114/35/a1594.pdf?ts=29.08.2017%2015:09:20> [retrieved on 20210317]

## Beschreibung

Zur Erfassung und Beurteilung eines Gesundheitszustandes eines Untersuchungsobjektes werden zunehmend umfangreiche Bilddatensätze einer interessierenden Körperregion des UntersuchungsobAnmjektes akquiriert, bspw. mittels Computertomographie, Magnetresonanztomographie, C-Bogen-Röntgengerät oder dergleichen. Diese Datensätze werden im Hinblick auf unterschiedliche medizinische Fragestellungen interpretiert bzw. befundet. Ein akquirierter Bilddatensatz kann, je nach medizinischer Fragestellung und/oder in Abhängigkeit der untersuchten Körperregion eine Bildserie mit bis zu mehreren tausend Einzelbildern umfassen. Damit beschränkt sich eine Befundung schon längst nicht mehr auf die Beurteilung eines dargestellten anatomischen Zustandes anhand einer medizinischen Bildaufnahme, wie es bspw. noch bei der Befundung anhand eines klassischen Röntgenbildes der Fall war. Insbesondere findet somit die Befundung zunehmend nicht mehr gleichzeitig zum Zeitpunkt der Bilderzeugung statt, sondern muss zumindest außerhalb der Notfallmedizin als zeitintensiver und von der Bilddatenakquise losgelöster Prozess angesehen werden. Insofern erlebt die radiologische Befundung zusammen mit der technologischen Weiterentwicklung der medizinischen Bilddatenakquise-Technologie einen erheblichen Entwicklungsschub. Tendenzen dieser Entwicklung umfassen die Errichtung von gro-ßen radiologischen Befundungseinheiten bzw. Befundungszentren mit mehreren parallelen Befundungsarbeitsplätzen, auch Workstations genannt, die entweder lokal in einem Gesundheitszentrum oder einem Krankenhaus oder zentral angesiedelt sind, wobei in letzterem Fall die radiologische Befundungseinheit als Dienstleister für mehrere Krankenhäuser oder eine Krankenhauskette fungieren kann. Tendenzen dieser Entwicklung umfassen weiter eine internationale Standardisierung der radiologischen Befundungsberichte, sodass ein Befundbericht unabhängig von Land, Region und/oder befundendem Radiologen einheitlich verständlich gelesen werden kann und sich für die Integration in eine elektronische und damit im Grunde überall verfügbare Patientenakte eignet.

Ein aktueller Standard-Prozess sieht bspw. folgendermaßen aus: die Radiologie eines Krankenhauses erhält eine medizinische Anfrage bzgl. eines Patienten XY aus einer nichtradiologischen Abteilung, in der Fachsprache auch Referrer genannt, bspw. der Kardiologie oder der Onkologie, oder der Pathologie. Nachdem der Patient XY bspw. mittels MRT oder CT oder ähnlichem gescannt bzw. untersucht wurde, interpretiert ein Radiologe die erfassten Bilddaten (in der Regel DICOM-Bilder) zu dem Patienten XY und diktiert parallel zur Betrachtung der Bilder klinische Befunde in einen radiologischen Befundbericht. Der Befundbericht umfasst die Gesamtheit aller aus den Bilddaten entnommenen medizinischen Erkenntnisse. Der Befundbericht, auch Report genannt, wird dann zusammen mit den zugrunde liegenden Bilddaten als bspw. sogenannte DICOM-Studie in einem lokalen Archiv-System, bspw. einem PACS (Picture Archiving and Communication System) abrufbar gespeichert. Zudem wird der Befundbericht, und zwar ohne die Bilddaten, an den Referrer gesandt. Auf die zusätzliche Übertragung der Bilddaten wird zum einem aus technischen Gründen verzichtet, da allein das zu übertragende Datenvolumen eine übliche Krankenhausnetz-Infrastruktur überlasten würde. Zum anderen fehlt auf Seiten der anfragenden medizinischen Abteilung regelmäßig die nötige Kenntnis und/oder Zeit für eine sachgemäße Interpretation der Bilddaten. Entsprechend geht bei der Kommunikation des Befundungsergebnisses zwischen Befundungseinheit und Referrer regelmäßig eine große Menge veranschaulichender, bildlicher Zusatzinformation verloren.

Die WO 2003/ 071 779 A1 offenbart ein System und ein Verfahren zum Erzeugen einer sequentiellen Anzeige von medizinischen Bilddaten. Dazu werden eine Vielzahl von medizinischen Bilddaten erfasst, in Gruppen sortiert und gruppenweise sequentiell in einer Endlosfilmschleife angezeigt.

Demgegenüber ist es Aufgabe der vorliegenden Erfindung, Mittel zur Anreicherung des Informationsgehaltes eines Befundberichtes bereit zu stellen, die weder spezielle Befundungskenntnisse seitens eines Referrers noch erhöhte Kapazitäten einer Krankenhausnetz-Infrastruktur erfordern.

Diese Aufgabe wird gelöst durch ein Verfahren zum Erzeugen eines Cinematic Rendering Kurzfilms basierend auf einem medizinischen Bilddatensatz und einem radiologischen Befundbericht, durch eine entsprechende Recheneinheit, entsprechendes Computerprogramm, entsprechenden computerlesbaren Datenträger sowie einen entsprechenden Befundungsarbeitsplatz gemäß den unabhängigen Ansprüchen. Bevorzugte und/oder alternative, vorteilhafte Ausgestaltungsvarianten sind Gegenstand der abhängigen Ansprüche.

Nachstehend wird die erfindungsgemäße Lösung der Aufgabe in Bezug auf das beanspruchte Verfahren als auch in Bezug auf beanspruchte Vorrichtungen beschrieben. Hierbei erwähnte Merkmale, Vorteile oder alternative Ausführungsformen sind ebenso auch auf die anderen beanspruchten Gegenstände zu übertragen und umgekehrt. Mit anderen Worten können gegenständliche Ansprüche (die beispielsweise auf ein Verfahren gerichtet sind) auch mit Merkmalen, die in Zusammenhang mit einer der Vorrichtungen beschrieben oder beansprucht sind, weitergebildet sein. Die entsprechenden funktionalen Merkmale des Verfahrens werden dabei durch entsprechende gegenständliche Module oder Einheiten ausgebildet.

Die vorliegende Erfindung betrifft in einem ersten Aspekt ein Verfahren zum Erzeugen eines Cinematic Rendering Kurzfilms. Das Erzeugen des Cinematic Rendering Kurzfilms basiert dabei auf
- einem medizinischen Bilddatensatz umfassend eine Bildserie, wobei der Bilddatensatz eine interessierende Körperregion eines Untersuchungsobjektes darstellt, und
- einem radiologischen Befundbericht, wobei der radiologische Befundbericht wenigstens einen klinischen Befund bezüglich der mittels Bilddatensatz dargestellten interessierenden Körperregion umfasst.

Das Verfahren umfasst eine Vielzahl von Schritten. In einem ersten Schritt erfolgt ein Auswählen eines klinischen Befundes des Befundberichts. In einem zweiten Schritt erfolgt ein Auswählen einer Teilbildserie des Bilddatensatzes basierend auf dem ausgewählten klinischen Befund. In einem dritten Schritt erfolgt ein Bestimmen eines Parametersatzes für einen Cinematic Rendering Algorithmus, wobei der Parametersatz Information zu bestimmten Protokollparametern und/oder anatomischen Strukturen innerhalb der abgebildeten, interessierenden Körperregion berücksichtigt. In einem vierten Schritt erfolgt ein Erzeugen eines Cinematic Rendering Kurzfilms durch Anwenden des Cinematic Rendering Algorithmus auf die ausgewählte Teilbildserie unter Verwendung des Parametersatzes. Bevorzugt erfolgt das Bestimmen des Parametersatzes unter Berücksichtigung des ausgewählten klinischen Befundes, der zugrunde liegenden, von einem Referrer an eine Radiologie übertragene, medizinische Fragestellung und/oder den in der ausgewählten Teilbildserie dargestellten anatomischen Strukturen innerhalb der interessierenden Körperregion, wie bestimmte Organe oder Gewebe. Die Fragestellung gibt bspw. Aufschluss über bestimmte Protokollparameter der Bilddatenakquise und damit über den Informationsgehalt der Bildserie, die dargestellte anatomische Struktur, bspw. Knochen oder Weichteilgewebe erfordern für eine vergleichbare Bild- bzw. Videoqualität jeweils unterschiedliche Einstellungen beim Cinematic Rendering. Derart kann folglich die Konfiguration des Cinematic Rendering Algorithmus auf die individuellen Gegebenheiten des untersuchten Patienten angepasst und eine besonders vorteilhafte Visualisierung bzw. Darstellung der anatomischen Struktur, bspw. einer befundeten Läsion, in dem Kurzfilm erzielt werden.

Erfindungsgemäß ist also vorgesehen, dass im Rahmen einer Befundung in einer Befundungseinheit komplexe, klinische, visuelle Zusatzinformation auf einfache und anschauliche Weise für einen Betrachter, insbesondere einen Mediziner außerhalb des radiologischen Fachbereichs, aufbereitet und neben einem Befundbericht zur Verfügung gestellt werden kann. Zu diesem Zweck wird medizinische Bildinformation, die in einem einer Befundung zugrunde liegenden medizinischen Bilddatensatz enthalten ist, unter Berücksichtigung eines ebenfalls daraus abgeleiteten Befundes in einen Cinematic Rendering Kurzfilm übertragen bzw. überführt. Durch die Auswahl bzw. das Bestimmen eines Parametersatzes, bevorzugt in Abhängigkeit des ausgewählten klinischen Befundes, der zugrunde liegenden, medizinische Fragestellung und/oder den in der ausgewählten Teilbildserie dargestellten anatomischen Strukturen wird ein Cinematic Rendering Algorithmus erfindungsgemäß so vorkonfiguriert, dass der Cinematic Rendering Kurzfilm den ausgewählten Befund bzw. eine dem Befund entsprechende anatomische Situation bzw. einen Gesundheitszustand oder Krankheitszustand eindeutig entnehmbar ist, selbst für einen ungeübten Betrachter. Der erfindungsgemäße Kurzfilm kann vorteilhaft einem Referrer, bspw. einem Neurologen oder einem Kardiologen, zusammen mit oder getrennt von dem Befundbericht zur Verfügung gestellt werden.

Im Folgenden wird ohne Beschränkung der Allgemeinheit von einem Patienten als Untersuchungsobjekt ausgegangen, wobei es sich meist um einen Menschen handelt. Grundsätzlich kann der Patient ein beliebiges Lebewesen oder ein Gewebe sein. Im Folgenden werden daher die beiden Begriffe "Untersuchungsobjekt" und "Patient" synonym verwendet. Das Untersuchungsobjekt kann alternativ eine Pflanze oder ein nicht-lebender Gegenstand, z.B. ein historisches Artefakt oder dergleichen sein.

Eine interessierende Körperregion im Sinne der Erfindung entspricht einem mittels beliebiger medizinischer Bildgebungsanlage abgebildeten (Teil-)Bereich, des Untersuchungsobjektes, insbesondere einer bestimmten Körperregion wie z.B. Kopf, Thorax, Hüfte, Unterschenkel, etc. Bei der interessierenden Körperregion handelt es sich insbesondere um eine symptomrelevante Körperregion. In diesem Fall werden Bilddaten nur bezüglich dieses (Teil-)Bereichs aufgenommen, die interessierende Körperregion kann aber auch das gesamte Untersuchungsobjekt umfassen. In diesem Fall erfasst die medizinische Bildgebungsanlage Bilddaten zu dem gesamten Körper des Untersuchungsobjektes.

Unter einem medizinischen Bilddatensatz soll im Sinne der Erfindung ein Satz von Bilddaten verstanden werden, der infolge einer medizinischen Fragestellung mit einer medizinischen Bildgebungsanlage bzgl. der interessierenden Körperregion erfasst wurde. Der Bilddatensatz liegt erfindungsgemäß im Bildraum bzw. im Ortsraum vor. Der Bilddatensatz kann als dreidimensionaler oder vierdimensionaler Bilddatensatz ausgebildet sein. Ein dreidimensionaler Bilddatensatz umfasst dabei drei Raumdimensionen. Ein vierdimensionaler Bilddatensatz umfasst zu den drei Raumdimensionen auch eine Zeitdimension. Mit anderen Worten kann der Bilddatensatz eine Bildserie mit wenigstens zwei Bildelementen umfassen, wobei einzelne Bildelemente bzw. Bilder der Bildserie im Wesentlichen gleichzeitig erfasst werden und einen räumlichen Versatz zu einander aufweisen und/oder einzelne Bildelemente der Bildserie einen zeitlichen Versatz zu einander aufweisen, bspw., wenn ein Kontrastmittelfluss oder eine Kontrastmittelanreicherung in einem bestimmten Gewebe innerhalb der interessierenden Körperregion oder die Funktion des Herzens dargestellt werden soll. Dabei können einzelne Bildelemente mittels bekannter Interpolationstechniken bspw. aus übrigen Bildelementen erzeugt worden sein. Ein typischer medizinischer Bilddatensatz umfasst bspw. mehrere hundert bis mehrere tausend Einzelbildelemente.

Eine Teilbildserie des Bilddatensatzes umfasst erfindungsgemäß eine Teilmenge von zusammenhängenden Bildelementen der Bildserie des Bilddatensatzes, welche im Einzelfall die gesamte Bildserie umfassen kann. Die Teilbildserie umfasst mindestens so viele einzelne Bildelemente, dass sie sich für die erfindungsgemäße Erzeugung eines Cinematic Rendering Kurzfilms eignet.

Ein Cinematic Rendering Kurzfilm ist in diesem Zusammenhang als zeitliche Abfolge von Einzel-Videoelementen, also als Kurzfilm bzw. Video-Clip mit kurzer Laufzeit, bspw. wenige Sekunden, bspw. 5 - 30 Sekunden, zu verstehen. Andere, insbesondere längere Laufzeiten sind ebenfalls möglich. Ein Cinematic Rendering Kurzfilm ist ferner dadurch gekennzeichnet, dass er mittels eines Cinematic Rendering Verfahrens bzw. Algorithmus erstellt wurde. Einzel-Videoelemente entsprechen jeweils einem gerenderten Frame bzw. Time Frame des Kurzfilms, ein Einzel-Videoelement kann insbesondere einem Einzelbildelement der Teilbildserie entsprechen, bspw. wenn der medizinische Bilddatensatz eine zeitliche Komponente umfasst. Der Cinematic Rendering Kurzfilm kann in verschiedenen Formaten, bspw. als DICOM Secondary Capture Element, als DICOM Video Stream (MPEG-4 AVC), als Animated GIF Element oder MPEG Stream, ausgebildet sein. Insbesondere ist vorgesehen, dass ein Cinematic Rendering Kurzfilm kompatibel und integrierbar in einen Workflow eines Krankenhaus-Informationssystem (HIS) oder ein Radiologie-Informationssystem (RIS), typischerweise entsprechend dem FHIR oder HL7 Standard ist.

Ein Cinematic Rendering Algorithmus im Sinne der Erfindung ist zu verstehen als neuartiges Nachbearbeitungs(Post Processing)-Verfahren für dreidimensionale Volumenbilddatensätze, wie sie bspw. mittels CT oder MRT gewonnen werden. Mittels Cinematic Rendering kann eine besonders natürliche bzw. photo-realistische Darstellung einer in den Bilddaten dargestellten, interessierenden Körperregion erzeugt werden. Cinematic Rendering eignet sich insbesondere zur Visualisierung von anatomischen Strukturen hoher Dichte bzw. mit hohem Kontrast, wie bspw. Knochengewebe oder kontrast-verstärkten Blutgefäßen. Cinematic Rendering Techniken berücksichtigen bei der Berechnung eines Pixeleintrags eines gerenderten Bildes, das Licht aus allen beliebigen Richtungen entlang eines Lichtstrahls gestreut werden kann. Dabei kommen typischerweise Monte Carlo Simulationen zum Einsatz, um zufallsbasiert eine Lichtstrahlenbündel mit günstiger räumlicher Verteilung zu ermitteln, welche dann bei der Strahlverfolgung berücksichtigt werden. Ein finales, gerendertes Bild ergibt sich typischerweise aus einer Mittelwertbildung einer Vielzahl von Monte-Carlo-Proben mit jeweils anderer Strahlverteilung und Streustrahlrichtungen. Bei der Erzeugung eines Cinematic Rendering Kurzfilms wird der Cinematic Rendering Algorithmus auf jedes einzelne Bildelement bzw. die Teilbildserie angewandt. Insbesondere kann beim Cinematic Rendering in dem Kurzfilm durch Änderung einer Betrachterperspektive bzw. der Position einer virtuellen Kamera relativ zu der dargestellten, interessierenden Körperregion ein Eindruck erzeugt werden, als würde sich der Betrachter um die interessierende Körperregion herum oder durch sie hindurch bewegen. Der Cinematic Rendering Algorithmus kann vorteilhaft ein Caching von Lichtstrahlreflektionen umfassen, um eine schnelle, insbesondere störungs- bzw. unterbrechungsarme Vorschau-Darstellung oder Ansicht des Cinematic Rendering Kurzfilms zu erzeugen. Derart wird dem Nutzer ermöglicht, schnell und vorab zu entscheiden, ob der Cinematic Rendering Kurzfilm oder besser gesagt jeweils die ausgewählte Teilbildserie und/oder der bestimmte Parametersatz bestmöglich auf den ausgewählten Befund abgestimmt ist, und ggf. Anpassungen vorzunehmen, bevor der Cinematic Rendering Kurzfilm final erstellt wird.

Der Parametersatzes des Cinematic Rendering Algorithmus umfasst wenigstens einen, in der Regel aber mehrere Parameter zur Konfiguration des Cinematic Renderings. In einem bevorzugten Ausführungsbeispiel der Erfindung umfasst der Parametersatz wenigstens einen Parameter aus der Gruppe der folgenden Cinematic Rendering Parameter: Beleuchtungsmodell, Zooming, Panning, Clipping, Transferfunktion, Kameraposition, Kameratrajektorie. Das Beleuchtungsmodell umfasst verschiedene Konfigurationen bzgl. der bei der Berechnung verwendeten künstlichen Lichtquelle, wie bspw. ihre Position in bzw. auf einem Objekt innerhalb der interessierenden Körperregion oder außerhalb desselben oder ihre Trajektorie, wenn sich die Position der Beleuchtungsquelle im Verlauf des Cinematic Rendering Kurzfilms ändern soll. Bei der künstlichen Lichtquelle kann es sich um eine virtuelle Lampe oder einen virtuellen Strahler handeln.

Bei einem radiologischen Befundbericht im Sinne der Erfindung handelt es sich um einen Bericht in Form eines elektronischen Dokuments, der als Ergebnis einer radiologischen Befundung entsteht. Form für bzw. Medium zur Archivierung und/oder Verteilung des Befundberichts sind nicht festgelegt. Jedoch ist der DICOM Standard eine inzwischen weit verbreitete Option, in der der Befundbericht als DICOM Structured Report archiviert und kommuniziert wird. Alternativ oder zusätzlich kann ein Befundbericht auch in Form eines HL7 Reports vorliegen, welcher in einem Krankenhausinformationssystem (HIS/KIS) abgespeichert werden kann. Es kann vorgesehen sein, dass ein DICOM Structured Report mittels HL7 CDA Konvertierungsverfahren in einen HL7 Report umgewandelt wird und umgekehrt. Vorgaben in Bezug auf seine Gliederung sowie inhaltliche Komponenten sind normiert. Ein Befundbericht umfasst mindestens Angaben zum Identifikation des Patienten und zu der durchgeführten Untersuchung. Ferner umfasst der Befundbericht auch wenigstens einen medizinischen Inhalt, der mindestens klinische Angaben, eine Fragestellung und eine Wertung, zusammengefasst einen klinischen Befund, beinhalten muss. Ein Befundbericht sollte idealerweise vollständig sein, d.h. er bezieht sich nur auf den zugehörigen und bspw. zusammen mit dem Befundbericht als DICOM Studie archivierten Bilddatensatz, die Bewertung der klinischen Angaben geht auf die formulierte Fragestellung ein, und andere auf den Bilddaten des Bilddatensatzes erkennbare Befunde müssen ebenfalls bewertet werden. Die Struktur des Befundberichtes ist idealerweise auch normiert, bspw. um eine Integration in eine elektronische Patientenakte zu ermöglichen. Zum Beispiel kann ein klinischer Befund textural strukturiert sein. Daneben können ergänzende multimediale Darstellungsformen erzeugt werden, die beispielsweise dynamische Visualisierungen von Befundbestandteilen zulassen.

Vorteilhaft kann erfindungsgemäß der erzeugte Cinematic Rendering Kurzfilm zusammen mit einem radiologischen Befundbericht und dem korrespondierenden Bilddatensatz als DICOM-Studie archiviert bzw. abgespeichert werden.

In einer bevorzugten Ausführungsvariante der Erfindung umfasst das Verfahren auch folgenden Schritt:
- Einfügen in den Cinematic Rendering Kurzfilm eines Direktzugriffselements, welches einen direkten Zugriff aus dem Cinematic Rendering Kurzfilm auf den klinischen Befund im Befundungsbericht erlaubt.

Diese Ausführungsvariante stellt eine Verknüpfung bzw. Verbindung zwischen dem Cinematic Rendering Kurzfilm und dem klinischen Befund her. Das Direktzugriffselement kann bspw. als Bildinhalt in einen oder eine Reihe von zusammenhängenden Frames bzw. Einzel-Videoelementen des Kurzfilms ausgebildet sein, bspw. als Einblendung eines Buttons oder als interaktiver Schriftzug. Andere Ausgestaltungen sind ebenfalls denkbar. Alternativ kann ein Direktzugriffselement als eine interaktive Markierung, bspw. ein Pfeil oder eine Linie innerhalb einer zusätzlich zu dem Cinematic Rendering Kurzfilm angezeigten Zeitachse über den Verlauf des Kurzfilms ausgebildet sein. Daneben sind andere Ausgestaltungen des Direktzugriffselements denkbar. Allen Ausgestaltungen ist gemein, dass eine Betätigung bzw. Aktivierung des Direktzugriffselements durch einen Nutzer, hier ein Betrachter, bspw. ein Mediziner außerhalb der Radiologie, der radiologische Befundungsbericht für den Nutzer angezeigt wird. Insbesondere wird der ausgewählte Befund des Befundberichts, auf den sich der Cinematic Rendering Kurzfilm bezieht, angezeigt. Insofern wird zusätzlich und ggf. zeitgleich zu dem klinischen Befund dem Betrachter veranschaulichender Bildinformation zu dem im Befund beschriebenen Gesundheitszustand bzw. Krankheitszustand zur Verfügung gestellt.

Es ist ferner vorgesehen, dass das Verfahren auch folgenden Schritt umfasst:
- Einfügen in den Befundungsbericht eines Direktzugriffselements, welches einen direkten Zugriff von einem klinischen Befund des Befundungsberichts auf den Cinematic Rendering Kurzfilm erlaubt.

Dies stellt eine Verknüpfung bzw. Verlinkung zwischen dem Cinematic Rendering Kurzfilm und dem klinischen Befund her, aber dieses Mal ausgehend von dem ausgewählten klinischen Befund in Richtung des Cinematic Rendering Kurzfilms. Das Direktzugriffselement in dem Befundbericht kann bspw. als interaktive Referenz bzw. Verlinkung auf den Speicherort des Cinematic Rendering Kurzfilms ausgestaltet sein. Das Direktzugriffselement kann bspw. als Referenz-Teilpfad ausgebildet sein, wenn der Cinematic Rendering Kurzfilm lokal, bspw. in einem PACS eines Krankenhauses archiviert ist, oder aber als vollständiger Referenz-Pfad auf einen Speicherort, wenn der Cinematic Rendering Kurzfilm bspw. zentral auf einem Cloud-Server abgelegt ist. Alternativ kann der Cinematic Rendering Kurzfilm direkt als Direktzugriffselement in den Befundungsbericht bezüglich des ausgewählten klinischen Befunds eingebettet werden, bspw. als DICOM Secondary Capture Objekt. Auch in diesem Ausführungsbeispiel ist vorgesehen, dass bei Aktivierung bzw. Betätigung des Direktzugriffselements in dem Befundungsbericht durch einen Nutzer der Cinematic Rendering Kurzfilm automatisch angezeigt, bzw. abgespielt wird, bspw. mittels einer geeigneten Abspiel-Software, die bei Betätigung des Direktzugriffselements ebenfalls mit aufgerufen wird. Insofern wird einem Nutzer, wieder ein Betrachter, der den ausgewählten klinischen Befund des Befundungsberichts liest bzw. analysiert, auf Wunsch instantan eine zusätzliche visuelle Veranschaulichung der dem Befund zugrunde liegenden anatomische Struktur bzw. Situation dargestellt.

Zusammengefasst stellt die vorliegende Erfindung also eine uni-direktionale und besonders bevorzugt eine bi-direktionale Schnittstelle zwischen einem klinischen Befund und einem Cinematic Rendering Kurzfilm her, wodurch wechselseitig der Gehalt an Information bzgl. eines Befunds oder Kurzfilms jeweils wechselseitig vorteilhaft und anschaulich ergänzt wird.

In einer anderen Ausführungsvariante der Erfindung wird eine Position des Direktzugriffselements in dem Cinematic Rendering Kurzfilm derart gewählt, dass eine Darstellung des Direktzugriffelements mit einer dem ausgewählten klinischen Befund zugrunde liegenden Darstellung der interessierenden Körperregion korreliert. Diese Ausführungsvariante sieht also insbesondere vor, dass das Direktzugriffselement in dem Cinematic Rendering Kurzfilm dann eingeblendet bzw. angezeigt wird, wenn ein dargestellter Bildinhalt bezüglich der interessierenden Körperregion bestmöglich den klinischen Befund veranschaulicht. Mit anderen Worten, wird das Direktzugriffselement derart in dem Cinematic Rendering Kurzfilm platziert bzw. positioniert bzw. angeordnet, dass das bzw. die Einzelframes bzw. Einzel-Videoelemente des Kurzfilms, die das Direktzugriffselement umfassen einen befundeten Krankheits- bzw. Gesundheitszustand bestmöglich veranschaulichen, bspw. die Größe und/oder Lage einer Läsion relativ zu einem anderen, insbesondere sensitiven Organ oder eine Funktionsschwäche bspw. einer Herzklappe.

In einer weiteren bevorzugten Ausführungsvariante wird die Position, also die zeitliche Position, des Direktzugriffselements in dem Cinematic Rendering Kurzfilm mittels eines maschinellen Lernverfahrens, einer statistischen Methode oder einem künstlichen neuronalen Netz bestimmt.

Unter maschinellen Lernverfahren kann man die künstliche Generierung von Wissen aus Erfahrung bezeichnen. Ein künstliches System lernt aus Beispielen in einer Trainingsphase und kann nach Beendigung der Trainingsphase verallgemeinern. Damit kann ein Algorithmus bzw. die zugrunde liegende Auswahl-Heuristik zum Bestimmen der optimalen Position für das Direktzugriffselement kontinuierlich angepasst werden. Die Verwendung maschineller Lernverfahren kann ein Erkennen von Mustern und Gesetzmäßigkeiten in den Trainingsdaten umfassen.

Eine statistische Methode kann beispielsweise Fuzzylogik, eine selbstorganisierende Karte, Stichprobenwiederholung (engl.: Resampling), Mustererkennung oder Support Vector Machine umfassen. Vorteilhaft kann auf der Basis der Erfahrungen ein Algorithmus zur Erkennung von Mustern oder Gesetzmä-ßigkeiten für eine Bestimmung einer geeigneten Position für das Direktzugriffselement verwendet werden.

Ein trainierter Algorithmus zum Bestimmen einer Position eines Direktzugriffselements in den Cinematic Rendering Kurzfilm kann ein neuronales Netz, ein System, eine Lerntransfermethode oder einen auf einer Datenbank oder auf mathematischen Funktionen oder Gleichungen basierten Algorithmus umfassen. Der trainierte Bestimmungsalgorithmus kann auf Basis bekannter Daten von historischen Bilddatensätzen und korrespondierenden Bildinhalten bezüglich der interessierenden Körperregion sowie den dazu gehörigen klinischen Befunden erstellt, verbessert oder angepasst werden. Als Input-Daten für das Training des Bestimmungsalgorithmus können bspw. Kombinationen der folgenden Informationen dienen: Messprotokolle alter Bilddatenaufnahmen, Informationen über die eingesetzte Bildgebungsanlage-Anlage, zu untersuchende Körperregion, eingesetzte Bildvorverarbeitungsschritte wie bspw. Segmentierungs- und/oder Artefaktkorrekturschritte, als für die Beurteilung eines Krankheits- bzw. Gesundheitszustandes als relevant eingestufte und in den Bilddaten entsprechend gekennzeichnete anatomische Strukturen, wie bspw. Läsionen oder ein bestimmter Gewebetyp, Informationen über den Parametersatz des Cinematic Rendering Algorithmus oder dergleichen. Diese Daten können bspw. aus Log-Dateien aller im Feld befindlichen Bildgebungsanlagen-Anlagen und/oder aus historischen DICOM-Studien jeweils umfassend wenigstens einen Cinematic Rendering Kurzfilm mit wenigstens einem Direktzugriffselement, lokal oder zentral archiviert, gewonnen werden. Für den Fall, dass es sich bei dem Bestimmungsalgorithmus um ein neuronales Netz handelt, kann als Trainingsverfahren das Überwachte Lernen zum Einsatz kommen, da zu den alten DICOM-Studien jeweils auch die bestimmten Positionen der Direktzugriffselemente als Input-Daten geliefert werden können. Eine für ein Direktzugriffselement bestimmte Position kann einer eindeutigen Relation oder einer über eine Wahrscheinlichkeit bedingten Relation entsprechen. Vorteilhaft können Muster oder Gesetzmäßigkeiten aus den historischen Trainingsdaten auf eine aktuelle Positionsbestimmung für ein Direktzugriffselement angewendet werden. Es kann im Einzelfall vorgesehen sein, dass die maschinell bestimmte Position für ein Direktzugriffselement eines Kurzfilms durch einen Nutzer angepasst, verändert bzw. korrigiert werden kann, bspw. wenn dem Nutzer eine andere Position vorteilhafter erscheint.

In einer weiteren bevorzugten Ausführungsvariante erfolgt das Auswählen des klinischen Befundes und/oder das Auswählen der Teilbildserie durch einen Nutzer. In dieser Ausführungsvariante der Erfindung ist vorgesehen, dass der Nutzer, in diesem Fall, ein befundender Radiologe, zumindest eine Auswahl des klinischen Befundes oder der Teilbildserie aus dem gesamten Bilddatensatz trifft, zu dem bzw. zu der er einen Cinematic Rendering Kurzfilm erstellen möchte. Entsprechend kann erfindungsgemäß vorgesehen sein, dass bspw. das Auswählen einer geeigneten Teilbildserie basierend auf einer Nutzereingabe bzgl. des klinischen Befundes automatisch erfolgt bzw. dem Nutzer zur Bestätigung oder Anpassung angezeigt wird. Alternativ kann vorgesehen sein, dass zu jedem klinischen Befund eines Befundberichts automatisch eine Teilbildserie des Bilddatensatzes dem Nutzer zur Auswahl bzw. zur Anpassung angezeigt wird. Eine automatische Auswahl einer Teilbildserie basiert dabei bspw. auf einer Analyse der in den Einzelbildern der Bildserie des Bilddatensatzes dargestellten Inhalte, anatomischen Strukturen, insbesondere nach wenigstens einem Nachbearbeitungsschritt wie einer Segmentierung, einer Rauschkorrektur und/oder einer Bewegungsartefaktkorrektur. In alternativen Ausführungsformen erfolgt wenigstens einer der beiden Auswahlschritte automatisch ohne Nutzer-Input.

In einer weiteren Ausführungsvariante der Erfindung erfolgt das Bestimmen des Parametersatzes unter Berücksichtigung des ausgewählten klinischen Befundes und/oder der ausgewählten Teilbildserie mittels eines maschinellen Lernverfahrens, einer statistischen Methode oder einem künstlichen neuronalen Netz. Bezüglich der Realisierung der verschiedenen, alternativen Varianten gilt das bereits gesagte.

Ein trainierte Bestimmungsalgorithmus kann auf Basis bekannter Daten von historischen Bilddatensätzen und korrespondierenden Cinematic Rendering Filmen, insbesondere Kurzfilmen, bezüglich der interessierenden Körperregion sowie den dazu gehörigen klinischen Befunden erstellt, verbessert oder angepasst werden. Als Input-Daten für das Training des Bestimmungsalgorithmus können bspw. Kombinationen der folgenden Informationen dienen: Messprotokolle alter Bilddatenaufnahmen, Informationen über die eingesetzte Bildgebungsanlage, zu untersuchende Körperregion, eingesetzte Bildvorverarbeitungsschritte wie bspw. Segmentierungsschritte, Rauschunterdrückung und/oder Bewegungsartefaktkorrekturen, als für die Beurteilung eines Krankheits- bzw. Gesundheitszustandes als relevant eingestufte und in den Bilddaten entsprechend gekennzeichnete anatomische Strukturen, Informationen über den Parametersatz alter Cinematic Rendering Algorithmen und Informationen über den zum Einsatz kommenden Cinematic Rendering Algorithmus. Diese Daten können bspw. aus Log-Dateien aller im Feld befindlichen Bildgebungsanlagen-Anlagen und/oder aus historischen DICOM-Studien jeweils umfassend wenigstens einen Cinematic Rendering Film bzw., lokal oder zentral archiviert, gewonnen werden. Für den Fall, dass es sich bei dem Bestimmungsalgorithmus um ein neuronales Netz handelt, kann als Trainingsverfahren das Überwachte Lernen zum Einsatz kommen, da zu den alten DICOM-Studien jeweils auch die jeweils verwendeten Parametersätze als Input-Daten geliefert werden können. Ein Parametersatz für einen Cinematic Rendering Algorithmus kann einer eindeutigen Relation oder einer über eine Wahrscheinlichkeit bedingten Relation entsprechen. Vorteilhaft können Muster oder Gesetzmäßigkeiten aus den historischen Trainingsdaten auf eine aktuelle Parametersatzbestimmung angewendet werden. Es kann im Einzelfall vorgesehen sein, dass ein maschinell bestimmter Parametersatz durch einen Nutzer angepasst, verändert bzw. korrigiert werden kann, bspw. wenn dem Nutzer eine andere Konfiguration des Cinematic Rendering Algorithmus vorteilhafter erscheint.

Alternativ kann der Parametersatz bspw. in Abhängigkeit eines ausgewählten klinischen Befundes, der in dem Bilddatensatz abgebildeten, interessierenden Körperregion, dem verwendeten Messprotokoll und/oder dergleichen default-mäßig vorgegeben sein.

Die Erfindung betrifft in einem anderen Aspekt eine Recheneinheit zum Erzeugen eines Cinematic Rendering Kurzfilms basierend auf
- einem medizinischen Bilddatensatz umfassend eine Bildserie, wobei der Bilddatensatz eine interessierende Körperregion eines Untersuchungsobjektes darstellt, und
- einem radiologischen Befundbericht, wobei der radiologische Befundbericht wenigstens einen klinischen Befund bezüglich der mittels Bilddatensatz dargestellten interessierenden Körperregion umfasst.

Die Recheneinheit weist erfindungsgemäß Mittel zum Durchführen eines erfindungsgemäßen Verfahrens auf.

In einer bevorzugten Ausführungsvariante der Recheneinheit sind ihre Mittel zum Durchführen des Verfahrens wenigstens teilweise Server-seitig angeordnet. Mit anderen Worten ist wenigstens eines ihrer Mittel server-seitig, also bspw. in der Cloud angeordnet. Dies hat den Vorteil, dass das zentral angeordnete Mittel von mehreren, lokal an verschiedenen Positionen angeordneten Recheneinheiten verwendet werden kann. Dies ist ressourcenschonend und effizient.

Besonders bevorzugt erfolgt der Schritt des Erzeugens des Cinematic Rendering Kurzfilme mittels eines Cloud-basierten Services zentral in der Cloud. In diesem Zusammenhang kann erfindungsgemäß vorgesehen sein, dass vor einer Übertragung der ausgewählten Teilbildserie und eines ausgewählten Parametersatzes für das Cinematic Rendering eine Anonymisierung der Daten im Hinblick auf Patienten-Identifikationsdaten erfolgt. Dazu kann bspw. eine Texterkennung und/oder eine OCR (optical character recognition)-Methode auf die zu übertragenden Daten angewandt werden und entsprechende Patientenidentifikationsdaten, entfernt, geschwärzt, überblendet und/oder anonymisiert werden.

In einem anderen Aspekt betrifft die Erfindung ein Computerprogramm mit Programmcode, um ein erfindungsgemäßes Verfahren durchzuführen, wenn das Computerprogramm auf einem Computer ausgeführt wird.

In einem weiteren Aspekt betrifft die Erfindung einen computerlesbarer Datenträger mit Programmcode eines Computerprogramms, um ein erfindungsgemäßes Verfahren durchzuführen, wenn das Computerprogramm auf einem Computer ausgeführt wird.

In einem letzten Aspekt betrifft die Erfindung einen Befundungsarbeitsplatz zum Erzeugen eines Cinematic Rendering Kurzfilms basierend auf
- einem medizinischen Bilddatensatz umfassend eine Bildserie, wobei der Bilddatensatz eine interessierende Körperregion eines Untersuchungsobjektes darstellt, und
- einem radiologischen Befundbericht, wobei der radiologische Befundbericht wenigstens einen klinischen Befund bezüglich der mittels Bilddatensatz dargestellten interessierenden Körperregion umfasst.

Dieser Befundungsarbeitsplatz weist eine erfindungsgemäße Recheneinheit auf. Ein Befundungsarbeitsplatz umfasst bevorzugt eine Eingabe- und Ausgabeeinheit. Die Ausgabeeinheit dient zur Anzeige von Bildinformation, von Befundungsberichten und/oder Cinematic Rendering Kurzfilmen. Die Ausgabeeinheit umfasst zu diesem Zweck wenigstens einen Anzeigebildschirm. Gemeinsam mit der Eingabeeinheit erfolgt eine Interaktion mit einem Nutzer, hier einem Radiologen, der eine Befundung eines Bilddatensatzes an dem Befundungsarbeitsplatz durchführt. Über die Eingabeeinheit kann der Nutzer Eingaben machen, bspw. bzgl. einer Auswahl einer Teilbildserie oder ein gewünschten Parametersatzes. Die Auswahl oder aber Auswahloptionen können dem Nutzer per Ausgabeeinheit angezeigt werden. Der Befundungsarbeitsplatz ist bspw. in einer Radiologie-Einheit angeordnet, neben einer Vielzahl weiterer Befundungsarbeitsplätzen. Der Befundungsarbeitsplatz kann lokal in einem Krankenhaus, oder auch zentral in einer eigenständigen Einheit ausgebildet sein, bspw. einem Radiologie-Zentrum, welches für mehrere Krankenhäuser parallel Befundungsdienstleistungen ausführt. Insbesondere kann ein Befundungsarbeitsplatz auch mobil ausgebildet sein, bspw. kann eine Eingabe- und Ausgabeeinheit als tragbares Tablet ausgebildet sein und die Recheneinheit in dieser Variante als zentrale Recheneinheit, wobei sich die Ein- und Ausgabeeinheit damit zur Datenkommunikation verbinden kann.

Die oben beschriebenen Eigenschaften, Merkmale und Vorteile dieser Erfindung sowie die Art und Weise, wie diese erreicht werden, werden klarer und deutlicher verständlich im Zusammenhang mit der folgenden Beschreibung der Ausführungsbeispiele, die im Zusammenhang mit den Zeichnungen näher erläutert werden. Durch diese Beschreibung erfolgt keine Beschränkung der Erfindung auf diese Ausführungsbeispiele. In verschiedenen Figuren sind gleiche Komponenten mit identischen Bezugszeichen versehen. Die Figuren sind in der Regel nicht maßstäblich. Es zeigen:
- FIG 1: eine Ansicht eines Systems umfassend einen erfindungsgemäßen Befundungsarbeitsplatz sowie eine erfindungsgemäße Recheneinheit gemäß einer Ausführungsform der vorliegenden Erfindung,
- FIG 2: eine schematische Darstellung eines erfindungsgemäßen Verfahrens gemäß einem Ausführungsbeispiel der vorliegenden Erfindung,
- FIG 3: eine Darstellung eines erfindungsgemäßen Cinematic Rendering Kurzfilms samt Direktzugriffselement in einem Ausführungsbeispiel der vorliegenden Erfindung, und
- FIG 4: eine Darstellung eines erfindungsgemäßen Befundberichts samt Direktzugriffselement in einem Ausführungsbeispiel der vorliegenden Erfindung.

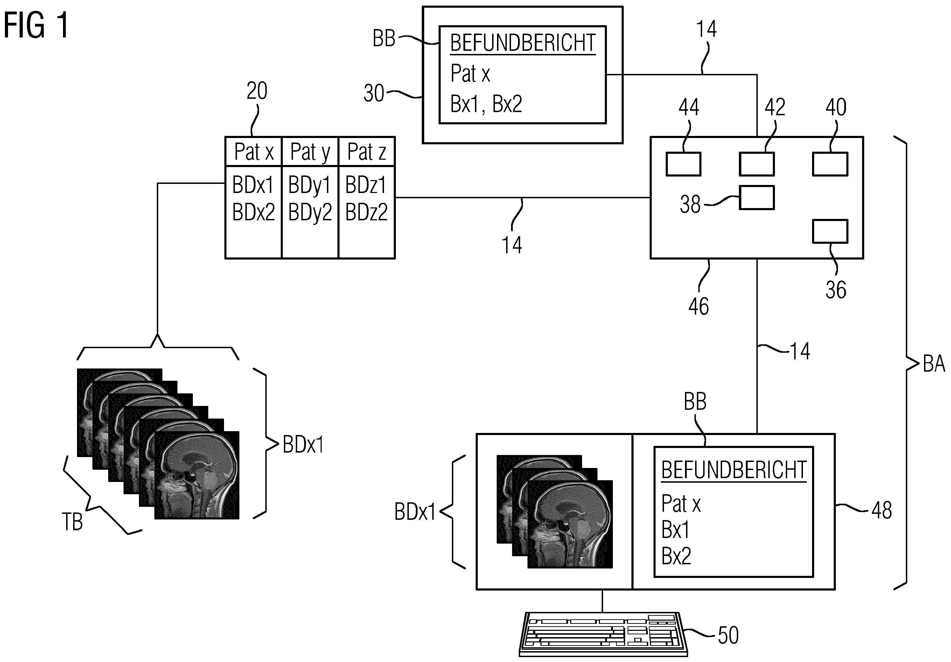

**Figur 1** zeigt ein System zur Speicherung, Ver- bzw. Bearbeitung, Übertragung und/oder zur Anzeige von medizinischen Bilddaten, bspw. einem CT-Bilddatensatz oder einem MRT-Bilddatensatz. Ein vergleichbares System kann bspw. in einem Krankenhaus oder einem beliebigen medizinischen Versorgungszentrum angeordnet sein. Einzelne Einheiten, Module bzw. Komponenten des Systems können lokal, in dem medizinischen Versorgungszentrum oder zentral an einem anderen Standort oder virtuell in der Cloud angeordnet sein. Das System umfasst eine Ausgabeeinheit 48 und eine Eingabeeinheit 50. Das System umfasst ferner eine erfindungsgemäße Recheneinheit 46. Die Recheneinheit 46 und die Ausgabe- und Eingabeeinheit 48, 50 stehen über eine Verbindung 14 in Datenkommunikation. Die genannten Komponenten bilden zusammen einen erfindungsgemäßen Befundungsarbeitsplatz BA. Insbesondere der erfindungsgemäße Befundungsarbeitsplatz BA ist zentral bzw. abgelegen angeordnet, bspw. dann wenn ein medizinisches Versorgungszentrum Teleradiologie als Dienstleistung in Anspruch nimmt. Bei der Teleradiologie wird radiologisches Bildmaterial an einen entfernten Ort übertragen, um dort durch einen fachkundigen Arzt ausgewertet zu werden. Der Ort der Befundung und der Ort der Untersuchung sind folglich entkoppelt und die Kommunikation erfolgt bspw. mittels sicherer, elektronischer Datenübertragung(z.B. Internet).

Die Ausgabe- und Eingabeeinheit 48, 50 dienen einem Informationsaustausch bzw. einer Interaktion mit einem Nutzer, hier ein Radiologe bzw. ein befundender Arzt. Die Ausgabeeinheit 48 dient beispielsweise zur graphischen Anzeige eines medizinischen Bilddatensatzes und/oder eines erfindungsgemäßen Cinematic Rendering Kurzfilms KF für einen Nutzer. Die Ausgabeeinheit dient auch bspw. zur Anzeige von Auswahloptionen bezüglich eines Parametersatzes P für einen Cinematic Rendering Algorithmus oder bezüglich einer Teilbildserie, die der Erzeugung eines Cinematic Rendering Kurzfilmes KF als Basis dienen soll. Die Eingabeeinheit 50 dient beispielsweise der Bestätigung bzw. der Eingabe von Auswahlinformation durch den Nutzer oder Information bzgl. der Anzeige eines bestimmten Bilddatensatzes BD, radiologischen Befundberichts BB oder dergleichen. Über die Eingabeeinheit 48 kann ein Nutzer insbesondere auch einen klinischen Befund bzw. einen entsprechenden Befundbericht BB erstellen. Über die Eingabeeinheit 50 hat der Nutzer N insbesondere die Möglichkeit, in den Ablauf des erfindungsgemäßen Verfahrens einzugreifen bzw. diesen zu steuern. Bei der Ausgabeeinheit 48 kann es sich beispielsweise um wenigstens einen LCD-, Plasma- oder OLED-Bildschirm handeln. Bevorzugt sind mehrere Bildschirme, insbesondere zwei Bildschirme, umfasst, um eine zeitgleiche Anzeige von verschiedener Information, bspw. ein Bilddatensatz BD und ein zugehöriger radiologischer Befund sowie einen dazugehörigen Cinematic Rendering Kurzfilm KF anzeigen zu können. Es kann sich bei der Ausgabeeinheit 48 weiterhin um einen berührungsempfindlichen Bildschirm handeln, welcher auch als Eingabeeinheit 50 ausgebildet ist. Insbesondere ist die Ausgabeeinheit 48 als portable Ausgabeeinheit, bspw. Handy oder Tablet ausgebildet und insofern auch von Standort der Recheneinheit 46 entkoppelt, sodass eine Befundung bzw. das erfindungsgemäße Verfahren unabhängig vom Aufenthaltsort des fachkundigen Radiologen ausgeführt werden kann. Bei der Eingabeeinheit 50 handelt es sich beispielsweise um eine Tastatur, eine Maus, einen sogenannten "Touch-Screen" um ein Mikrofon zur Spracheingabe und/oder einen Fußschalter. Mikrofon und Fußschalter haben insbesondere den Vorteil, dass die Hände des Nutzers N frei bleiben. Die Eingabeeinheit 50 kann auch eingerichtet sein, um Bewegungen eines Benutzers zu erkennen und in entsprechende Befehle zu übersetzen.

Die Recheneinheit 46 ist ausgebildet, alle Schritte des Erfindungsgemäßen Verfahrens auszuführen. Dazu kann die Recheneinheit 46 verschiedene Module bzw. Einheiten umfassen. Insbesondere steht die Recheneinheit 46 zu diesem Zweck mit einer Bildspeichereinheit 20 sowie einer Befundspeichereinheit 30 über Verbindungen 14 in Datenaustausch. Die Bildspeichereinheit 20 kann als Picture Archiving and Communication System (PACS) ausgebildet sein und dient der digitalen Archivierung und Kommunikation von digitalen Bilddaten in Form von medizinischen Bilddatensätzen BD in einem Netzwerk. In der Bildspeichereinheit 20 können Bilddaten aller Modalitäten und verschiedener Patienten Pat X, Pat Y, Pat Z archiviert werden. Zu einem Patienten können mehrere Bilddatensätze BDX1 - BDZ1 entsprechend verschiedener Untersuchungen eines Patienten abgespeichert werden. Das PACS 20 ermöglicht einen schnellen, unkomplizierten Zugriff auf die Daten, schnelle Verfügbarkeit und Reproduzierbarkeit. Erfolgt eine Speicherung eines Befundberichts BB im DICOM Structured Report Format, kann auch dieser als Teil einer DICOM-Studie in der Bildspeichereinheit 20 archiviert werden. Die Befundspeichereinheit 30 kann bspw. als Radiologieinformationssystem (RIS) einer Radiologie eines Krankenhauses ausgebildet sein, aber auch als Krankenhausinformationssystem (HIS/KIS). Die Befundspeichereinheit 30 kümmert sich in erster Linie um die Verwaltung von Patientenstammdaten. Dazu kommuniziert es mit anderen Systemkomponenten. Die Kommunikation erfolgt meistens mittels HL7 Standard. Bspw. werden die Patientenstammdaten verwendet, um Bilddatensätze mit entsprechenden Metadaten auszustatten. Darüber hinaus dient die Befundspeichereinheit 30 auch der digitalen Archivierung von radiologischen Befundberichten BB umfassend wenigstens einen klinischen Befund Bx1, Bx2, die für einen bestimmten Patienten, bspw. Patient Pat X vorliegen. Insbesondere erfolgt die Archivierung von Befundberichten BB in der Befundspeichereinheit 30 im HL7 Format. Über die bereits angesprochenen Patienten-Metadaten lassen sich die archivierten Befundberichte BB zu den jeweiligen Bilddatensätzen BD im PACS eindeutig zuordnen.

Die Recheneinheit 46 kann vorteilhaft eine Empfangseinheit 44 umfassen, welche jegliche Auswahl-bzw. Eingabebefehle, bspw. bzgl. einer Teilbildserie TB, bzgl. einer Position eines Direktzugriffselements DZE in einem Cinematic Rendering Kurzfilm KF oder einem radiologischen Befundbericht BB und/oder bzgl. eines klinischen Befundes Bx1, Bx2 eines Befundberichts BB, eines Nutzers empfängt und für eine weitere Berücksichtigung aufbereitet. Darüber hinaus ist die Empfangseinheit 44 auch eingerichtet, über Verbindung 14 Bilddatensätze BD und/oder Befundberichte BB von Bildspeichereinheit 20 bzw. Befundspeichereinheit 30 zu empfangen. Die Recheneinheit 46 umfasst ferner eine Bestimmungseinheit 42 zur Bestimmung eines Parametersatzes P für einen Cinematic Rendering Algorithmus. Dazu kann die Bestimmungseinheit 42 Informationen bzgl. des ausgewählten klinischen Befunds Bx1, Bx2, der einer Untersuchung zugrunde liegende medizinischen Fragestellung und damit Information zu bestimmten Protokollparametern und/oder anatomische Strukturen innerhalb der abgebildeten, interessierenden Körperregion berücksichtigen. Insbesondere kann die Bestimmungseinheit 42 eingerichtet sein, unter Anwendung eines maschinellen Lernverfahrens den Parametersatz P zu bestimmen. Die Recheneinheit 46 umfasst auch eine zweite Bestimmungseinheit 40 zum Bestimmen einer Position eines Direktzugriffselements in einem Cinematic Rendering Kurzfilm. Diese ist insbesondere eingerichtet, ein ausgewählte Teilbildserie bzw. deren Bildinhalt auszuwerten und zu analysieren, derart, dass sie Einzelbildelemente identifiziert, deren Bildinhalt bestmöglich zu einem ausgewählten Befund Bx1 passt bzw. korreliert. Darüber hinaus umfasst die Recheneinheit 46 auch eine Render-Einheit 38, die eingerichtet ist, aus allen durch die übrigen Einheiten zur Verfügung gestellten Informationen unter Anwendung eines Cinematic Rendering Algorithmus ein Cinematic Rendering Kurzfilm zu erstellen. Insbesondere die Render-Einheit 38 kann als eigenständige Einheit ausgelagert sein und bspw. in Form eines Web-basierten Services durch einen Cloud-Server ausgebildet sein.

Im Übrigen können alle genannten Einheiten der Recheneinheit 46 körperlich und/oder funktional mit einander verbunden oder getrennt sein.

Die Recheneinheit 46 kann mit einem computerlesbaren Datenträger zusammenwirken, insbesondere um durch ein Computerprogramm mit Programmcode ein erfindungsgemäßes Verfahren durchzuführen. Weiterhin kann das Computerprogramm auf dem maschinenlesbaren Träger abrufbar gespeichert sein. Insbesondere kann es sich bei dem maschinenlesbaren Träger um eine CD, DVD, Blu-Ray Disc, einen Memory-Stick oder eine Festplatte handeln. Die Recheneinheit 46 kann in Form von Hard- oder in Form von Software ausgebildet sein. Beispielsweise ist die Recheneinheit 46 als ein sogenanntes FPGA (Akronym für das englischsprachige "Field Programmable Gate Array") ausgebildet oder umfasst eine arithmetische Logikeinheit.

In einem Speicher 36 der Recheneinheit 46 ist wenigstens ein Computerprogramm gespeichert, welches alle Verfahrensschritte des erfindungsgemäßen Verfahrens durchführt, wenn das Computerprogramm auf dem Computer ausgeführt wird. Das Computerprogramm zur Ausführung der Verfahrensschritte des erfindungsgemäßen Verfahrens umfasst Programmcode. Weiterhin kann das Computerprogramm als ausführbare Datei ausgebildet sein und/oder auf einem anderen Rechensystem. Beispielsweise das gezeigte System ausgelegt sein, dass die Recheneinheit 46 das Computerprogramm zum Ausführen des erfindungsgemäßen Verfahrens über ein Intranet oder über das Internet in seinen internen Arbeitsspeicher lädt.

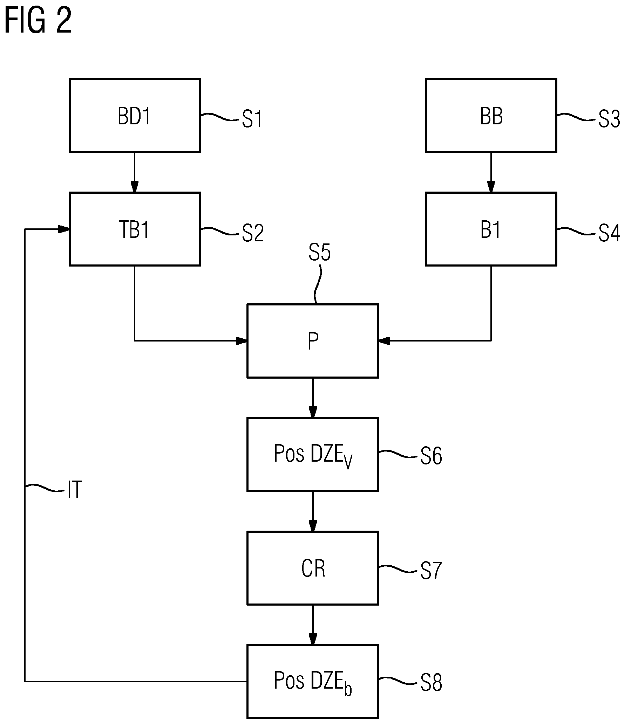

**Figur 2** zeigt ein Ablaufschema eines erfindungsgemäßen Verfahrens in einem Ausführungsbeispiel der Erfindung. Das Verfahren wird bspw. an einem erfindungsgemäßen Befundungsarbeitsplatz BA ausgeführt. An solchen Befundungsarbeitsplätzen BA werden bspw. Graustufenbilder und/oder Bilder in Falschfarben in 2D, 3D oder 4D (zeitabhängige Darstellungen, bspw. Aktivitätsänderung einer interessierenden Körperregion oder Darstellung von Körperflüssigkeitsbewegungen), die mit Hilfe einer Aufnahmetechnik wie bspw. CT, MRT, PET oder ähnlichem aufgenommen wurden, ausgewertet, also bewertet und befundet. Das heißt die Bilddaten werden nach pathologischen Mustern abgesucht. Außerdem müssen Charakteristika der Bilddaten analysiert, vermessen und interpretiert werden. Befunde werden diktiert und entweder zur weiteren Verarbeitung weitergeleitet oder mittels einer entsprechenden Software direkt in den Computer eingespeist.

Bevorzugt wird das erfindungsgemäße Verfahren direkt während bzw. nach der Erstellung eines Befundberichts BB zu einem darin umfassten klinischen Befund Bx1, Bx2 durchgeführt. Das erfindungsgemäße Verfahren umfasst eine Vielzahl von Schritten, die im Folgenden einzeln näher beschrieben werden:

In einem ersten Schritt S1 wird ein Bilddatensatz BDx1 mittels Ausgabeeinheit 48 dem Nutzer angezeigt. Parallel dazu wird in einem Schritt S3 dem Nutzer auch ein auf dem Bilddatensatz basierender Befundbericht BB angezeigt. Wie in Figur 1 dargestellt. In einem Schritt S4 wählt der Nutzer, bspw. über eine Markierung mittels Maus einen in dem Befundbericht BB enthaltenen Befund Bx1 aus, den er mittels eines Cinematic Rendering Kurzfilms KF zusätzlich bildlich für einen Betrachter in der Empfängerkette des Befundberichts BB veranschaulichen möchte. Zur oder während der Erzeugung des Befundes Bx1 kann der Nutzer N optional Bildbearbeitungs-Tools einer Befundungs-Applikation einsetzen. bspw. um Organe und/oder Blutgefäße zu segmentieren, Landmarken zu detektieren, eine Synchronisation von Bilddaten und Herzschlägen oder andere Vorverarbeitungsschritte durchzuführen. Bevorzugt können die Ergebnisse dieser Vorverarbeitungsschritte in dem Bilddatensatz BD1 durch entsprechende Markierungen, Einfärbungen oder zusätzliche Einblendungen nachvollziehbar festgehalten sein. In einem Schritt S2 kann der Nutzer nun basierend auf dem ausgewählten Befund Bx1 eine Teilbildserie TB aus dem Bilddatensatz BDx1 auswählen, die für die Erstellung des Cinematic Rendering Kurzfilms KF verwendet werden sollen. Bspw. kann diese Auswahl durch Markierung von Einzelbildelementen des Bilddatensatzes BDx1 mittels Maus erfolgen. Alternativ kann der Nutzer über eine Eingabe von Relativkoordinaten zu der abgebildeten interessierenden Körperregion eine Teilbildserie TB definieren. Insbesondere bei vier-dimensionalen Bilddaten BD kann eine Auswahl auch über die Angabe von zu Einzelbildelementen gehörigen Aufnahmezeiten den Beginn und das Ende der Teilbildserie festlegen. Die Teilbildserie kann den gesamten Bilddatensatz oder aber nur eine beliebige Teilmenge an Einzelbildelementen des Bilddatensatzes BD umfassen. In einem Schritt S5 erfolgt die Bestimmung eines Parametersatzes P für einen Cinematic Rendering Algorithmus. Bevorzugt erfolgt die Bestimmung automatisch durch die Recheneinheit 46. Insbesondere kann vorgesehen sein, dass der Parametersatz P sich aus Default-Einstellungen ergibt, die in Abhängigkeit von bspw. dem verwendeten Cinematic Rendering Algorithmus, im Befund Bx1 befindlichen Schlagwörtern, Werten von bei der Bilddatenakquise eingesetzten Protokollparametern und/oder von in der Teilbildserie TB umfasster Bildinformation, bspw. eine bestimmte Gewebeart und/oder eine bestimmte Körperregion wie bspw. Kopf oder Thorax, gemäß einer Look-Up-Tabelle im Speicher 36 der Recheneinheit 46 ergeben. Das ist dann vorteilhaft, wenn der Nutzer unzureichend Kenntnis von Cinematic Rendering Techniken hat und mit einer individuellen Auswahl bzw. Anpassung von Parametern überfordert wäre. Alternativ und/oder zusätzlich kann vorgesehen sein, dass in diesem Schritt der Nutzer eine Anpassung wenigstens eines default-mäßig eingestellten Parameters des Parametersatzes P mittels Eingabeeinheit 50 vornimmt. Alternativ dazu kann vorgesehen sein, dass ein Nutzer alle Parameter des Parametersatzes direkt selber einstellt. Insbesondere kann in dieser Variante vorgesehen sein, dass der Nutzer Parameter bzgl. einer virtuellen Lichtquelle bzw. Kamera einstellt, bspw. kann er eine Auswahl zwischen einer statischen Lichtquelle innerhalb der gerenderten interessierenden Körperregion, einer statischen, an einem festen Bezugspunkt angeordneten Lichtquelle außerhalb der gerenderten interessierenden Körperregion und/oder einer Lichtquelle wählen, deren Position an die Position der virtuellen Kamera gebunden ist. Darüber hinaus kann vorgesehen sein, dass der Nutzer folgende Parameter selber einstellt: Transfer Funktion, Zooming, Panning, Clipping, usw.

Alternativ dazu kann ein maschinelles Lernverfahren oder dergleichen zur Bestimmung des Parametersatzes für das Cinematic Rendering zum Einsatz kommen. In einem weiteren Verfahrensschritt S6 erfolgt eine Bestimmung einer Position für ein Direktzugriffselement DZEv für den Cinematic Rendering Kurzfilm KF. Der Schritt kann auch umfassen, die Ausgestaltungsform des Direktzugriffselements DZEv festzulegen. Das Direktzugriffselement DZEv kann bspw. als Einblendung und/oder Markierung in einem parallel zum Abspielen des Kurzfilms KF angezeigten Zeitstrahl ausgebildet sein. Es stellt einen Direktzugriff aus dem Kurzfilm KF auf den entsprechenden Befund Bx1 des Befundberichtes BB her. Genauer gesagt verknüpft das Direktzugriffselement DZEv eine bestimmte Stelle bzw. bestimmte Einzelvideo-Elemente des Kurzfilms KF umfassend eine besonders deutliche Veranschaulichung der dem Befund Bx1 zugrunde liegenden anatomischen Situation und/oder dem entsprechenden Gesundheitszustand mit dem Befund Bx1. Auch dieser Schritt kann entweder durch manuelle Auswahl durch den Nutzer realisiert sein, der bspw. in der ausgewählten Teilbildserie entsprechende Einzelbildelemente markiert, die beim Rendering mit dem Direktzugriffselement DZEv versehen werden sollen. Alternativ kann dieser Schritt erneut mittels maschinellem Lernverfahren (s.o.) oder dergleichen ausgeführt werden, wobei insbesondere verschiedene Infos aus den Bilddaten und/oder dem Befund und/oder dem den Bilddaten zugrunde liegenden Messprotokoll und/oder alten Cinematic Rendering Kurzfilmen mit Direktzugriffselementen DZEv bzw. deren Positionen im Kurzfilm berücksichtigt werden können.

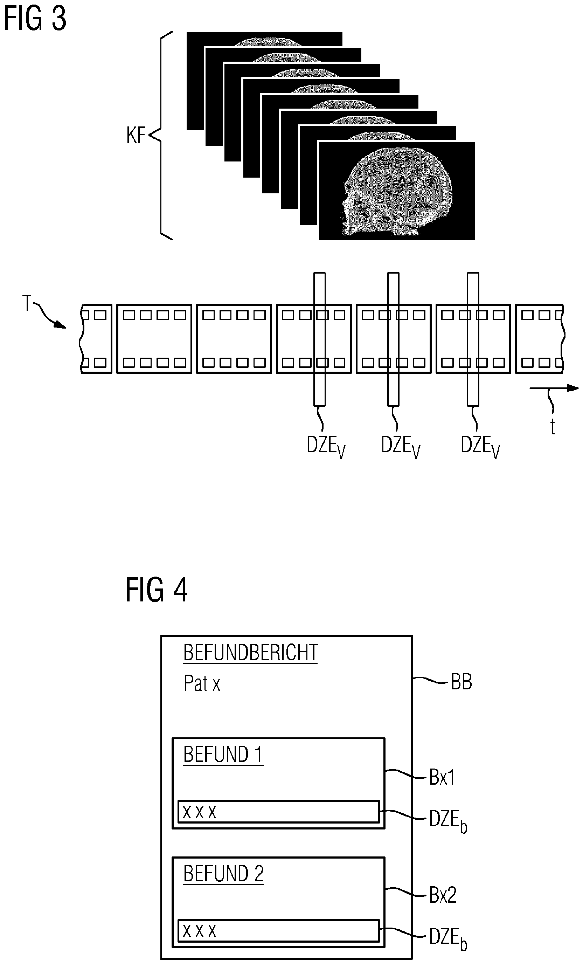

Ein Ausführungsbeispiel eines erfindungsgemäßen Cinematic Rendering Kurzfilms KF samt Direktzugriffselement DZEv ist in **Figur 3** veranschaulicht. Hier ist das Direktzugriffselement DZEv als interaktiver Markierungsbalken über allen Einzelvideoelementen des Kurzfilms KF ausgebildet, welche parallel zum Abspielen des Kurzfilms KF auf einer Zeitskala T mit angezeigt werden

In einem weiteren Schritt S7 erfolgt das Cinematic Rendering basierend auf der ausgewählten Teilbildserie TB sowie dem bestimmten Parametersatz. Mit anderen Worten wird in Schritt S7 die Serie von Einzelbildelementen der Teilbildserie TB in eine Serie von Einzelvideo-Elementen des Cinematic Rendering Kurzfilms KF überführt. Der Cinematic Rendering Kurzfilm KF kann bspw. als Teil einer DICOM-Studie zusammen mit dem Bilddatensatz BDx1 in der Bildspeichereinheit 20 des Systems archiviert werden. Alternativ kann der Kurzfilm KF in der Cloud, bevorzugt anonymisiert, abgelegt werden. Alternative Speicherorte sind ebenfalls möglich. In einem weiteren, optionalen Schritt S8 erfolgt das Bestimmen einer Position und Ausgestaltung eines Direktzugriffselements DZEb, welches in den Befundbericht BB, bevorzugt nahe des bzw. in dem ausgewählten klinischen Befund Bx1 integriert wird und einen Direktzugriff aus dem Befundbericht BB auf den Cinematic Rendering Kurzfilm KF ermöglicht, sodass bei Betätigung des Direktzugriffselements DZEb beide Informationen, also der veranschaulichende, Kurzfilm KF und der zugrunde liegende Befund Bx1, parallel einem Betrachter zur Verfügung stehen. Das Direktzugriffselement DZEb kann in Abhängigkeit des Speicherorts des Kurzfilms KF bspw. als Teilpfad oder vollständiger Pfad ausgebildet sein, sofern der Kurzfilm KF lokal innerhalb des Systems abgelegt ist, das Direktzugriffselement DZEb kann aber auch als Web-Link ausgebildet sein, wenn der Kurzfilm KF in der Cloud abgespeichert wurde. Grundsätzlich kann jeder beliebige Referenz-Pfand in den Befundungsbericht BB integriert werden. Bevorzugt wird das Direktzugriffselement DZEv im Dokument-Baum eines strukturierten Befundberichts (Structured Report), bspw. als an sich bekanntes DICOM Attribut, gespeichert. Dadurch wird verhindert, dass das Direktzugriffselement DZEv beim Archivieren im PACS oder bei einer Konvertierung in eine HL7 CDA Format für den Einschluss in eine elektronische Patientenakte in der Befundspeichereinheit 30verloren gehen kann.

**Figur 4** zeigt eine Darstellung eines erfindungsgemäßen Befundberichts BB zu einem Patienten Pat X samt Direktzugriffselement DZEb in einem Ausführungsbeispiel der Erfindung. Der Befundbericht umfasst zwei Befunde Bx1 und Bx2, jeweils versehen mit einem Direktzugriffselement DZEb für einen jeweiligen Cinematic Rendering Kurzfilm KF.

Für Schulungszwecke und/oder die Kommunikation mit Medizinern außerhalb der Radiologie kann vorgesehen sein, einen an sich bekannten WEB-Link in einen Befundbericht BB zu integrieren, der direkt auf den zu einem Befund gehörige Cinematic Rendering Kurzfilm KF verweist, bspw. in folgender Form: http://intranet.someClinic.org/referenceRenderings/Stream:UID _proceeding_02-12-17/0:06:40/0:42:32

Es kann vorgesehen sein, dass der Nutzer N in einem weiteren Verfahrensschritt (nicht dargestellt) über die Eingabeeinheit 50 eingibt, wo und/oder in welcher Form bzw. in welchem Format der erzeugte Cinematic Rendering Kurzfilm KF abgespeichert werden soll.

Das erfindungsgemäße Verfahren kann in einer optionalen Wiederholschleife IT mehrfach durchlaufen werden, wobei für verschiedene Befunde Bx1, Bx2 eines Befundberichtes BB die Schritte S2 bis S8 basierend auf demselben Bilddatensatz BD1 durchlaufen werden können, um mehrere Cinematic Rendering Kurzfilme KF zu erzeugen.

Diese können zu einem Gesamtvideo kombiniert werden, wobei dann ein jeweiliges Direktzugriffselement DZEb aus einem der Befunde Bx1, Bx2 auf den dazu gehörigen Kurzfilm KF innerhalb des Gesamtvideos verweist. Ausgehend von dem Gesamtvideo kann eine Vielzahl von Direktzugriffselementen DZEv bestehen, die jeweils auf verschiedene Befunde eines oder verschiedener Befundberichte verweisen.

In einem Klinik-Alltag sind anspruchsvolle und komplexe klinische Fragestellungen an der Tagesordnung. Diese stellen auch hohe Anforderungen an eine klare und eindeutige Kommunikation zwischen Radiologie und anderen Klinik-Einheiten. Oft sind Bilddaten, die zusammen mit Befunden und entsprechenden Markierungen bzw. Kennzeichnungen in den Bilddaten zur Verfügung gestellt werden für weniger erfahrenes und/oder geschultes Personal nur schwer oder unzureichend nachzuvollziehen oder zu interpretieren. Eine gute und leicht verständliche Aufbereitung von unterstützender Bildinformation unter Ausnutzung neuester Cinematic Rendering Techniken entsprechend der vorliegenden Erfindung kann hier Abhilfe schaffen.

Ein möglicher klinischer Anwendungsfall der vorliegenden Erfindung ist bspw. eine Anfrage an eine Radiologie bezüglich einer Funktionsstörung bzw. -beeinträchtigung eines Ventrikels eines Patienten. Auf Grundlage aller verfügbaren klinischen Informationen zu dem Krankheitszustand des Patienten muss eine geeignete und insbesondere schonende Behandlungsart aus einer Vielzahl von möglichen Therapieoptionen ermittelt werden. Unter anderem kommen invasive Behandlungsverfahren aber auch eine Medikation in Betracht. Um die am besten geeignete Therapieform zu ermitteln, werden auch Ergebnisse von Belastungstests berücksichtigt, insbesondere umfassend Aussagen zu Bio-Markern wie bspw. dem natriuretischen Faktor Typ B. Eine eingängige und leicht verständliche Darstellung der anatomischen Gegebenheiten des Patienten durch einen erfindungsgemäßen 3D-Cinematic Rendering Kurzfilm basierend auf einem 4D-Bilddatensatz kann sowohl die Kommunikation des Befundes als auch die anschließende Wahl der Therapieform verbessern bzw. vereinfachen.

Ein anderer beispielhafter Anwendungsfall ist eine Anfrage an eine Radiologie bzgl. eines Patienten mit einem Aneurysma, bspw. im Kopf oder Abdomen. Ein Patient zeigt Symptome eines Blutstaus, auch hier gibt es eine Vielzahl von verschiedenen Behandlungsoptionen, bspw. einen endovaskulären Verschluss des betroffenen Blutgefäßes oder eine chirurgische Behandlung. Anhand eines erfindungsgemäßen 3D Cinematic Rendering Kurzfilms können Veränderungen bzw. Verbesserungen eines Gesundheitszustandes von zu verschiedenen Zeitpunkten aufgenommenen Bilddatensätzen leicht verständlich veranschaulicht und für einen Betrachter außerhalb der Radiologie verfügbar gemacht werden.

Neben der Erstellung eines Befundberichts ggf. umfassend einzelne Bildelemente mit Kennzeichnungen bzw. Markierungen zur Veranschaulichung eines Befundes kann einem Radiologen über die zur Befundung verwendete Software-Applikation die Möglichkeit eingeräumt werden, die in dem Bilddatensatz enthaltene Bildinformation in einen 3D Cinematic Rendering Kurzfilm zu überführen und diesen neben dem Befundbericht an den Referrer zu leiten.

Die erfindungsgemäße Rendering-Funktion kann als eines von einer Vielzahl von Tools bzw. Werkzeugen einer Bildanzeige-, Bildbearbeitungs- und/oder Befundungssoftware in Form eine Anwendung bzw. Applikation ausgebildet sein, mit der ein Radiologie im Rahmen einer Befundung interagiert. Dabei kann insbesondere vorgesehen sein, dass der Radiologe manuell Renderingparamater wie bspw. das Beleuchtungsmodell, Zooming, Panning, Transfer Funktion, Kamera Position, Kameratrajektorie, Blickwinkel oder dergleichen auswählen bzw. einstellen kann. Alternativ kann die Cinematic Rendering Funktion als automatisch ablaufender Prozessschritt der Befundung vorgesehen sein, den der Radiologe nach einer Auswahl einer Teilbildserie starten oder aber überspringen kann. In dieser Variante erzeugt die Applikation unter Verwendung von voreingestellten oder automatisch ermittelten Parametern, wenigstens einen Cinematic Rendering Kurzfilm für wenigstens einen Befund. Besonders bevorzugt ermittelt das Rendering Tool spezifische Rendering Settings bzw. Parameter basierend auf einer Datenanalyse bereits bestehender, alter Cinematic Rendering Kurzfilme. Insbesondere dann, wenn die Befundungsanwendung Befundungsrichtlinien und/oder Vorlagen für Befundberichte verwendet, kann vorgesehen sein, dass die Befundungsanwendung den Radiologen anleitet bzw. ihm empfiehlt, zu welchem Befund ein abhängig von der Befundart bzw. dem Befundtyp ein Cinematic Rendering Kurzfilm erstellt werden sollte. Besonders bevorzugt ist die Ausführung von Schritt S7 des erfindungsgemäßen Verfahrens in Form eines Cloud-basierten Services auf einen zentralen Server ausgelagert, sodass die Cinematic Rendering Funktionalität nicht lokal auf einem Client, bspw. einem erfindungsgemäßen Befundungsarbeitsplatz BA bzw. in der Recheneinheit 46 angeordnet ist.

Wo noch nicht explizit geschehen, jedoch sinnvoll und im Sinne der Erfindung, können einzelne Ausführungsbeispiele, einzelne ihrer Teilaspekte oder Merkmale mit einander kombiniert bzw. ausgetauscht werden, ohne den Rahmen der hiesigen Erfindung zu verlassen. Mit Bezug zu einem Ausführungsbeispiel beschriebene Vorteile der Erfindung treffen ohne explizite Nennung, wo übertragbar, auch auf andere Ausführungsbeispiele zu.

## Patentansprüche

1. Verfahren zum Erzeugen eines Cinematic Rendering Kurzfilms (KF) basierend auf
- einem medizinischen Bilddatensatz (BDx1) umfassend eine Bildserie, wobei der Bilddatensatz eine interessierende Körperregion eines Untersuchungsobjektes (Pat X) darstellt, und
- einem radiologischen Befundbericht (BB), wobei der radiologische Befundbericht wenigstens einen klinischen Befund (BDx1) bezüglich der mittels Bilddatensatz dargestellten interessierenden Körperregion umfasst,
mit folgenden Schritten:
- Auswählen (S4) eines klinischen Befundes (Bx1) des Befundberichts,
- Auswählen (S2) einer Teilbildserie (TB) des Bilddatensatzes basierend auf dem ausgewählten klinischen Befund,
- Bestimmen (S5) eines Parametersatzes (P) für einen Cinematic Rendering Algorithmus, wobei der Parametersatz Information zu bestimmten Protokollparametern und/oder anatomischen Strukturen innerhalb der abgebildeten, interessierenden Körperregion berücksichtigt,
- Erzeugen (S7) eines Cinematic Rendering Kurzfilms durch Anwenden des Cinematic Rendering Algorithmus auf die ausgewählte Teilbildserie unter Verwendung des Parametersatzes, und
- Einfügen (S8) in den Befundungsbericht eines Direktzugriffselements (DZEb), welches einen direkten Zugriff von einem klinischen Befund des Befundungsberichts auf den Cinematic Rendering Kurzfilm erlaubt.

2. Verfahren nach Anspruch 1, welches auch folgenden Schritt umfasst:
- Einfügen (S6) in den Cinematic Rendering Kurzfilm eines Direktzugriffselements (DZEv), welches einen direkten Zugriff aus dem Cinematic Rendering Kurzfilm auf den klinischen Befund im Befundungsbericht erlaubt.

3. Verfahren nach einem der Ansprüche 1 oder 2, wobei eine Position des Direktzugriffselements in dem Cinematic Rendering Kurzfilm derart gewählt wird, dass eine Darstellung des Direktzugriffelements mit einer dem ausgewählten klinischen Befund zugrunde liegenden Darstellung der interessierenden Körperregion korreliert.

4. Verfahren nach Anspruch 3, wobei die Position des Direktzugriffselements in dem Cinematic Rendering Kurzfilm mittels eines maschinellen Lernverfahrens, einer statistischen Methode oder einem künstlichen neuronalen Netz bestimmt wird.

5. Verfahren nach einem der vorhergehenden Ansprüche, wobei das Auswählen des klinischen Befundes und/oder das Auswählen der Teilbildserie durch einen Nutzer (N) erfolgt.

6. Verfahren nach einem der vorhergehenden Ansprüche, wobei der Parametersatz wenigstens einen Parameter aus der Gruppe der folgenden Cinematic Rendering Parameter umfasst: Beleuchtungsmodell, Zooming, Panning, Clipping, Transferfunktion, Kameraposition, Kameratrajektorie.

7. Verfahren nach einem der vorhergehenden Ansprüche, wobei das Bestimmen des Parametersatzes unter Berücksichtigung des ausgewählten klinischen Befundes und/oder der ausgewählten Teilbildserie mittels eines maschinellen Lernverfahrens, einer statistischen Methode oder einem künstlichen neuronalen Netz erfolgt.

8. Recheneinheit (46) zum Erzeugen eines Cinematic Rendering Kurzfilms (KF) basierend auf
- einem medizinischen Bilddatensatz (BDx1) umfassend eine Bildserie, wobei der Bilddatensatz eine interessierende Körperregion eines Untersuchungsobjektes (Pat X) darstellt, und
- einem radiologischen Befundbericht (BB), wobei der radiologische Befundbericht wenigstens einen klinischen Befund (Bx1) bezüglich der mittels Bilddatensatz dargestellten interessierenden Körperregion umfasst,
aufweisend Mittel (36, 38, 40, 42, 44) zum Durchführen eines Verfahrens gemäß einem der Ansprüche 1 bis 7.

9. Recheneinheit gemäß Anspruch 8, deren Mittel zum Durchführen des Verfahrens wenigstens teilweise Server-seitig angeordnet sind.

10. Computerprogramm mit Programmcode, um das Verfahren nach einem der Ansprüche 1 bis 7 durchzuführen, wenn das Computerprogramm auf einem Computer ausgeführt wird.

11. Computerlesbarer Datenträger mit Programmcode eines Computerprogramms, um das Verfahren nach einem der Ansprüche 1 bis 7 durchzuführen, wenn das Computerprogramm auf einem Computer ausgeführt wird.

12. Befundungsarbeitsplatz (BA) zum Erzeugen eines Cinematic Rendering Kurzfilms (KF) basierend auf
- einem medizinischen Bilddatensatz (BDx1) umfassend eine Bildserie, wobei der Bilddatensatz eine interessierende Körperregion eines Untersuchungsobjektes (Pat X) darstellt, und
- einem radiologischen Befundbericht (BB), wobei der radiologische Befundbericht wenigstens einen klinischen Befund (Bx1) bezüglich der mittels Bilddatensatz dargestellten interessierenden Körperregion umfasst,
mit einer Recheneinheit (46) nach Anspruch 9.

## Claims

1. Method for generating a cinematic rendering short film (KF), based on
- a medical image data set (BDx1) comprising a series of images, wherein the image data set represents a body region of interest of an examination object (Pat X), and
- a radiological findings report (BB), wherein the radiological findings report comprises at least one clinical finding (BDx1) relating to the body region of interest represented by means of the image data set,
with the following steps:
- selecting (S4) a clinical finding (Bx1) of the findings report,
- selecting (S2) a partial image series (TB) of the image data set based on the selected clinical finding,
- determining (S5) a parameter set (P) for a cinematic rendering algorithm, wherein the parameter set takes into consideration information regarding particular protocol parameters and/or anatomical structures within the mapped body region of interest,
- generating (S7) a cinematic rendering short film by applying the cinematic rendering algorithm to the selected partial image series, using the parameter set, and
- inserting (S8) a direct access element (DZEb), which allows direct access of a clinical finding of the findings report to the cinematic rendering short film, into the findings report.

2. Method according to claim 1, which also comprises the following step:
- inserting (S6) a direct access element (DZEv), which allows direct access from the cinematic rendering short film to the clinical finding in the findings report, into the cinematic rendering short film.

3. Method according to one of claims 1 or 2, wherein a position of the direct access element in the cinematic rendering short film is chosen in such a manner that a representation of the direct access element correlates with a representation of the body region of interest underlying the selected clinical finding.

4. Method according to claim 3, wherein the position of the direct access element in the cinematic rendering short film is determined by means of a machine learning method, a statistical method or an artificial neural network.

5. Method according to one of the preceding claims, wherein the selecting of the clinical finding and/or the selecting of the partial image series takes place by way of a user (N).

6. Method according to one of the preceding claims, wherein the parameter set comprises at least one parameter from the group of the following cinematic rendering parameters:
lighting model, zooming, panning, clipping, transfer function, camera position, camera trajectory.

7. Method according to one of the preceding claims, wherein the determining of the parameter set takes place while taking into consideration the selected clinical finding and/or the selected partial image series by means of a machine learning method, a statistical method or an artificial neural network.

8. Computing unit (46) for generating a cinematic rendering short film (KF), based on
- a medical image data set (BDx1) comprising a series of images, wherein the image data set represents a body region of interest of an examination object (Pat X), and
- a radiological findings report (BB), wherein the radiological findings report comprises at least one clinical finding (Bx1) relating to the body region of interest represented by means of the image data set, having means (36, 38, 40, 42, 44) for performing a method according to one of claims 1 to 7.

9. Computing unit according to claim 8, of which the means for performing the method are arranged at least partially on the server side.

10. Computer program with program code, in order to perform the method according to one of claims 1 to 7, when the computer program is executed on a computer.

11. Computer-readable data carrier with program code of a computer program, in order to perform the method according to one of claims 1 to 7, when the computer program is executed on a computer.

12. Diagnostic workstation (BA) for generating a cinematic rendering short film (KF), based on
- a medical image data set (BDx1) comprising a series of images, wherein the image data set represents a body region of interest of an examination object (Pat X), and
- a radiological findings report (BB), wherein the radiological findings report comprises at least one clinical finding (Bx1) relating to the body region of interest represented by means of the image data set,
with a computing unit (46) according to claim 9.

## Revendications

1. Procédé de production d'un film (KF) court cinématique de rendu sur la base de
- un ensemble (BDx1) de données d'image médical comprenant une série d'images, dans lequel l'ensemble de données d'image représente une région intéressante du corps d'un objet (Pat X) en examen, et
- un rapport (BB) de constatation radiologique, dans lequel le rapport de constatation radiologique comprend au moins une constatation (BDx1) clinique se rapportant à la région du corps intéressante représentée au moyen de l'ensemble de données d'image,
comprenant les stades suivants :
- sélection (S4) d'une constatation (Bx1) clinique du rapport de constatation,
- sélection (S2) d'une série (TB) partielle d'image parmi l'ensemble de données d'image sur la base de la constatation clinique sélectionnée,
- détermination (S5) d'un ensemble (P) de paramètres pour un algorithme cinématique de rendu, dans lequel l'ensemble de paramètres prend en compte de l'information sur des paramètres de protocoles déterminés et/ou des structures anatomiques au sein de la région du corps intéressante représentée,
- production (S7) d'un film court cinématique de rendu par application de l'algorithme cinématique de rendu à la série partielle d'image sélectionnée en utilisant l'ensemble de paramètres, et
- insertion (S8) dans le rapport de constatation d'un élément (DZEb) d'accès direct, qui autorise un accès direct d'une constatation clinique du rapport de constatation au film court cinématique de rendu.

2. Procédé suivant la revendication 1, qui comprend également le stade suivant :
- insertion (S6) dans le film court cinématique de rendu d'un élément (DZEv) d'accès direct, qui autorise un accès direct, à partir du film court cinématique de rendu, à la constatation clinique du rapport de constatation.

3. Procédé suivant l'une des revendications 1 ou 2, dans lequel on sélectionne une position de l'élément d'accès direct dans le film court cinématique de rendu, de manière à corréler une représentation de l'élément d'accès direct à une représentation, à la base de la constatation clinique sélectionnée, de la région du corps intéressante.

4. Procédé suivant la revendication 3, dans lequel l'élément d'accès direct est déterminé, dans le film court cinématique de rendu, au moyen d'un procédé d'apprentissage automatique, d'une méthode statistique ou d'un réseau neuronal artificiel.

5. Procédé suivant l'une des revendications précédentes, dans lequel la sélection de la constatation clinique et/ou la sélection de la série partielle d'image s'effectue par un utilisateur (N).

6. Procédé suivant l'une des revendications précédentes, dans lequel l'ensemble de paramètres comprend au moins un paramètre choisi dans le groupe des paramètres cinématiques de rendu suivants : modèle d'éclairage, zooming, panning, clipping, fonction de transfert, position de la caméra, trajectoire de la caméra.

7. Procédé suivant l'une des revendications précédentes, dans lequel la détermination de l'ensemble de paramètres, en tenant compte de la constatation clinique sélectionnée et/ou de la série partielle d'images sélectionnée, s'effectue au moyen d'un procédé d'apprentissage automatique d'une méthode statistique ou d'un réseau neuronal artificiel.

8. Unité (46) informatique de production d'un film (KF) court cinématique de rendu reposant sur
- un ensemble (BDx1) de données d'image médical comprenant une série d'images, dans lequel l'ensemble de données d'image représente une région intéressante du corps d'un objet (Pat X) en examen, et
- un rapport (BB) de constatation radiologique, dans lequel le rapport de constatation radiologique comprend au moins une constatation (Bx1) clinique se rapportant à la région du corps intéressante représentée au moyen de l'ensemble de données d'image,
comportant des moyens (36, 38, 40, 42, 44) pour exécuter un procédé suivant l'une des revendications 1 à 7.

9. Unité informatique suivant la revendication 8, dont les moyens pour exécuter le procédé sont disposés en moins en partie du côté du serveur.

10. Programme d'ordinateur ayant un code de programme, pour effectuer le procédé suivant l'une des revendications 1 à 7, lorsque le programme d'ordinateur est exécuté sur un ordinateur.

11. Support de données, déchiffrable par ordinateur, ayant un code de programme d'ordinateur pour exécuter le procédé suivant l'une des revendications 1 à 7, lorsque le programme d'ordinateur est exécuté sur un ordinateur.

12. Emplacement (BA) de travail de constatation pour la production d'un film (KF) court cinématique de rendu sur la base de
- un ensemble (BDx1) de données d'image médicale comprenant une série d'images, dans lequel l'ensemble de données d'image représente une région intéressante du corps d'un objet (Pat X) en examen, et
- un rapport (BB) de constatation radiologique, dans lequel le rapport de constatation radiologique comprend au moins une constatation (Bx1) clinique se rapportant à la région du corps intéressante représentée au moyen de l'ensemble de données d'image,
comprenant une unité (46) informatique suivant la revendication 9.
